# EUROPEAN PATENT APPLICATION

(11) **EP 0 781 997 A1**
(43) Date of publication of application: **02.07.1997**
(21) Application number: 96402913.6
(22) Date of filing: 27.12.1996
(51) Int. Cl.: G01N 33/53, G01N 33/543, G01N 33/545, G01N 33/553, G01N 33/569, G01N 33/576, G01N 33/72

(54) **Instrument for simplified immunoassay and simplified immunoassay method using the instrument**

(30) Priority: 27.12.1995 JP 352064/95; 26.06.1996 JP 165526/96
(71) Applicant: FUJIREBIO Inc., Shinjuku-ku, Tokyo 163-07 (JP); AUBEX CORPORATION, Sumida-ku Tokyo 130 (JP)
(72) Inventor: Kakutani, Masami, Sagamihara-shi, Kanagawa-ken (JP); Sakuda, Ryutaro, Kamagaya-shi, Chiba-ken (JP); Yanagiya, Takayuki, Obihiro-shi, Hokkaido (JP); Kanai, Masahiro, Ohta-ku, Tokyo (JP)
(74) Representative: Gillard, Marie-Louise

(57) **Abstract**

An instrument for simplified immunoassay (1) for detecting the presence of at least one of an antigen or an antibody in a liquid specimen, includes a rod-shaped core member (2) which bears thereon at least one labeled antigen or antibody zone (X) including a labeled antigen or a labeled antibody, the labeled antigen and the labeled antibody respectively being capable of reacting with the antibody and the antigen in the liquid specimen, each forming a labeled antigen-antibody complex, and a detection zone (Y) disposed above the labeled antigen or antibody zone (X), the detection zone (Y) including an antigen or an antibody capable of reacting with the labeled antigen-antibody complex, and a cover member (3) which covers the outer peripheral surface of the rod-shaped core member (2), through which the labeled antigen or antibody zone (X) and the detection zone (Y) formed on the outer peripheral surface of the rod-shaped core member (2) are visible, with a capillary portion being provided between the rod-shaped core member (2) and the cover member (3), through which capillary portion the liquid specimen is carried upward from a base portion thereof so as to have the liquid specimen reach the labeled antigen or antibody zone (X) and then the detection zone (Y). A simplified immunoassay method using this instrument is provided.

## Description

### BACKGROUND OF THE INVENTION

### Field of the Invention

The present invention relates to an instrument for simplified immunoassay for detecting an antigen or an antibody in a specimen, and also to a simplified immunoassay method using the instrument, more particularly to an instrument for simplified immunoassay, for instance, for human immunodeficiency virus (hereinafter referred to as HIV), hepatitis B, hepatitis C, occult blood (hemoglobin in the feces), or pregnancy, and to a simplified immunoassay method using the instrument for such immunoassays.

### Discussion of Background

As simple methods for examining diseases in the digestive system such as cancer of the stomach and carcinoma of the colon, there are known a method of detecting the occult blood in the feces as disclosed in Japanese Laid-Open Patent Application 59-182367, and a method of detecting hemoglobin in the feces as disclosed in Japanese Laid-Open Patent Application 61-228951.

Varieties of instruments for collecting the feces for such examinations have been developed. In the above-mentioned Japanese Laid-Open Patent Application 59-182367, there is proposed a diagnosis instrument comprising a dip stick for attaching protein thereto, with at least part of the surface thereof being coated with a monoclonal antibody which has a specificity to human hemoglobin.

Japanese Laid-Open Utility Model Application 62-69160 discloses an instrument for collecting the feces, which comprises a dip stick in which a through-hole extending in the direction normal to the axis of the dip stick is formed near the tip of the dip stick. This instrument is used by inserting the tip of the dip stick into the feces so as to collect the feces in the through-hole or to attach the feces to a portion near the through-hole.

Japanese Laid-Open Patent Application 61-102941 discloses a collection rod with a cutaway portion or a concave portion.

Furthermore, Japanese Laid-Open Patent Application 61-228351 discloses a spoon for collecting the feces, with lattice-shaped grooves being formed on the surface thereof.

In the method of detecting the hemoglobin in the feces described in the above-mentioned Japanese Laid-Open Patent Application 59-182367, there is employed a feces collection container which comprises a feces collector and a liquid container which contains a buffer solution which is prepared so as to inhibit the activities of digestive enzymes in the feces. The hemoglobin in the feces is physically adsorbed on the surface of the feces collector and then specifically detected and assayed by use of an antihuman enzyme-labeled hemoglobin.

In the above-mentioned method which uses the feces collection container, it is indispensable that the hemoglobin on the collector be physically adsorbed thereto by the buffer solution in the liquid container, and thereafter the hemoglobin in the feces is assayed by a conventional enzyme immunoassay. Therefore, not only complicated examination operations, but also reagents for the enzyme immunoassay are required for the above assay. Thus, the above assay has not yet been simplified.

The method for detecting hemoglobin disclosed in the above-mentioned Japanese Laid-Open Patent Application 61-228951 also has similar problems to the above-mentioned problems.

### SUMMARY OF THE INVENTION

It is therefore an object of the present invention to provide a simplified immunoassay instrument with high reliability.

Another object of the present invention is to provide a simplified immunoassay instrument which is capable of constantly collecting a predetermined amount of a liquid specimen, without using a pipette, by changing a specimen collecting portion of the immunoassay instrument to be used, when detecting the presence or absence of a particular antigen or antibody in a liquid specimen, such as blood, serum, blood plasma, urine, saliva and expectoration; and which is capable of removing solid components and impurities such as non-specific components contained in the liquid specimen, by changing the shape, surface area and material of a capillary in the specimen collecting portion of the immunoassay instrument, thereby allowing the antigen or the antibody contained in the liquid specimen to react with a labeled antigen or a labeled antibody to bind them, and thus securing the reaction in a detection zone in the immunoassay instrument.

A further object of the present invention is to provide a simplified immunoassay method using the above-mentioned immunoassay instrument.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:

Fig. 1 is a partially cutaway perspective view of an example of an instrument for simplified immunoassay of the present invention.

Fig. 2 is a schematic cross-sectional view of the instrument for simplified immunoassay shown in Fig. 1.

Fig. 3 is a partially cutaway perspective view of another example of an instrument for simplified immunoassay of the present invention.

Fig. 4 is a schematic partially cutaway cross-sectional view of a liquid specimen collecting portion of the instrument for simplified immunoassay shown in Fig. 3.

Fig. 5 is a schematic partially cutaway perspective view of an example of a rod-shaped core member for use in the instrument for simplified immunoassay of the present invention.

Fig. 6 is a schematic partially cutaway perspective view of still another example of a rod-shaped core member for use in the instrument for simplified immunoassay of the present invention.

Fig. 7 is a schematic partially cutaway perspective view of a further example of a rod-shaped core member for use in the instrument for simplified immunoassay of the present invention.

Fig. 8 is a schematic partially cutaway perspective view of a further example of a rod-shaped core member for use in the instrument for simplified immunoassay of the present invention.

Fig. 9 is a schematic partially cutaway perspective view of a further example of a rod-shaped core member for use in the instrument for simplified immunoassay of the present invention.

### DESCRIPTION OF THE PREFERRED EMBODIMENTS

The instrument for simplified immunoassay of the present invention is for detecting an antigen or an antibody in a specimen.

Examples of specimens suitable for the instrument of the present invention are blood, serum, blood plasma, feces, urine, semen, saliva and expectoration.

An example of the instrument for simplified immunoassay of the present invention comprises a rod-shaped core member with a base portion and a top end portion, on the outer peripheral surface thereof, there being successively formed in the direction from the base portion toward the top end portion, (a) at least one labeled antigen or antibody zone comprising a labeled antigen or a labeled antibody, the labeled antigen being capable of reacting with the antibody in the liquid specimen, and the labeled antibody being capable of reacting with the antigen in the liquid specimen, each forming a labeled antigen-antibody complex, and (b) a detection zone which is disposed above the labeled antigen or antibody zone on the outer peripheral surface of the rod-shaped core member, the detection zone comprising an antigen or an antibody capable of reacting with the labeled antigen-antibody complex, and a cover member which covers the outer peripheral surface of the rod-shaped core member, through which the labeled antigen or antibody zone and the detection zone provided on the outer peripheral surface of the rod-shaped core member are visible, with a capillary portion being provided between the rod-shaped core member and the cover member, through which capillary portion the liquid specimen is carried upward in the direction from the base portion to the top end portion so as to have the liquid specimen to reach the labeled antigen or antibody zone and then the detection zone.

There are no particular restrictions with respect to the materials for the rod-shaped core member as long as the rod-shaped member has an appropriate thickness and length for the immunoassay. For instance, the rod-shaped core member may have a diameter of about 3.0 mm, and a length of about 60 to 70 mm.

The rod-shaped core member is preferably made of a synthetic resin, examples of such material being polypropyler (PP), polystyrene (PS), polyvinyl chloride (PVC), polyacetal (POM), and polyamide (PA).

It is preferable that the rod-shaped core member be white in color in order that the reaction in the detection zone can be clearly seen from the outside. However, the color of the rod-shaped core member is not necessarily limited to white, since the problem of the visibility of the reaction in the detection zone can be solved by appropriately selecting the color of a labeling material to be attached to the labeled antigen or labeled antibody in the labeled antigen or antibody zone. For instance, when the rod-shaped core member is black, titanium oxide (TiO₂) which is white in color can be used as a labeling material.

As mentioned above, on the outer peripheral surface thereof of the rod-shaped core member, there is provided at least one labeled antigen or antibody zone comprising a labeled antigen or a labeled antibody. The labeled antigen is capable of reacting with the antibody in the liquid specimen, and the labeled antibody is capable of reacting with the antigen in the liquid specimen, each forming a labeled antigen-antibody complex.

As a labeling material for forming the labeled antigen or the labeled antibody, for example, a metal colloid such as gold colloid, a color latex and an organic coloring material can be employed. Such labeling materials are for facilitating the visible inspection of the labeled antigen or antibody zone.

The labeled antigen or the labeled antibody can be provided in the form of a ring-shaped zone which encircles the outer peripheral surface of the rod-shaped core member.

As mentioned above, above the labeled antigen or antibody zone on the outer peripheral surface of the rod-shaped core member, there is disposed the detection zone which comprises an antigen or an antibody capable of reacting with the labeled antigen-antibody complex, so that when the antigen or the antibody is present in the liquid specimen, the presence of the antigen or the antibody is made visible.

Different antigens or antibodies are employed for the detection zone, depending upon examination subjects, but examples thereof are antigens or antibodies of HIV, hepatitis B, hepatitis C, occult blood, and other antigens or antibodies used for the diagnosis at emergency, and for the examination of pregnancy at home.

The antigen or antibody for the detection zone can be provided in the form of a ring-shaped zone encircling the outer peripheral surface of the rod-shaped core member.

In the instrument for simplified immunoassay of the present invention, there is provided the cover member which covers the outer peripheral surface of the rod-shaped core member, through which the labeled antigen or antibody zone and the detection zone provided on the outer peripheral surface of the rod-shaped core member are visible, with a capillary portion being provided between the rod-shaped core member and the cover member, through which capillary portion the liquid specimen is carried upward in the direction from the base portion to the top end portion so as to have the liquid specimen to reach the labeled antigen or antibody zone and then the detection zone.

In the above instrument for simplified immunoassay, the capillary portion may be a plurality of grooves formed along the inner surface of the cover member, which extends in the direction from the base portion to the top end portion of the rod-shaped core member.

There are no particular limitations to the form and the depth of the above-mentioned grooves formed in the inner surface of the cover member as long as the liquid specimen is carried upward by capillary action in the direction from the base portion to the top end portion so as to have the liquid specimen to reach the labeled antigen or antibody zone and then the detection zone.

However, it is preferable that the depth of the above-mentioned grooves formed in the inner surface of the cover member be in the range of 0.1 µm to 300 µm.

The assay time and the antigen-antibody reaction rate for this instrument can be controlled by adjusting the depth of the grooves.

Examples of the materials for the cover member are resins such as polypropylene (PP), polystyrene (PS), polyvinyl chloride (PVC), and ethylene vinyl acetate (EVA).

An example of the cover member is a cylindrical hollow member with an inner diameter which is slightly larger than the outer diameter of the rod-shaped core member, and with a number of grooves formed along the inner surface of the cover member, extending in the direction from the base portion to the top end portion of the rod-shaped core member so as to form capillaries between the inner surface of the cover member and the outer peripheral surface of the rod-shaped core member.

It is necessary that at least the above-mentioned labeled antigen or antibody zone, the above-mentioned detection zone, and a confirmation zone which will be explained later be visible through the cover member for the visual inspection thereof.

For this reason, it is not always necessary that the cover member be transparent or semitransparent in its entirety.

In the instrument for simplified immunoassay of the present invention, there may be provided a liquid specimen collecting portion for collecting the liquid specimen so as to carry the liquid specimen upward through the capillary portion from the base portion of the rod-shaped core member along the peripheral surface thereof, for instance at the base portion of the rod-shaped core member.

Another example of the liquid specimen collecting portion can be constructed as follows:

The above-mentioned cover member is extended downward beyond the base portion of the rod-shaped core member to form a space under the base portion of the rod-shaped core member in such a manner that the thus formed space is surrounded by the cover member, and the liquid specimen collecting portion is composed of a water-absorbing material, which is movably fitted into the space with a predetermined gap, for instance, of about 1 to 5 mm, between the base portion of the rod-shaped core member and a base end portion of the liquid *specimen* collecting portion. In order that the liquid specimen collecting portion can absorb a predetermined amount of the liquid specimen, when collecting the liquid specimen, the above-mentioned gap is maintained, but when the assay is started, the liquid specimen collecting portion is depressed so as to be fitted into the space and can be connected with the base portion of the rod-shaped core member, thereby the liquid specimen being carried from the liquid specimen collection portion upward through the capillary portion along the peripheral surface of the rod-shaped core member.

There are no limitations to the material for and the shape of the liquid specimen collecting portion as long as it is capable of collecting a predetermined amount of the liquid specimen, and removing solid components and impurities such as non-specific components contained in the liquid specimen.

The liquid specimen collecting portion may be in any shape, for example, in the shape of a circular cylinder or a circular cone.

Examples of the water-absorbing material for this liquid specimen collecting portion are an open-cell sponge material and a felt.

Furthermore, a confirmation zone for confirming the activity of the labeled antigen or the labeled antibody may be formed above the detection zone on the outer peripheral surface of the rod-shaped core member.

The confirmation zone comprises an antigen or an antibody which is capable of reacting and recognizing the labeled antibody or the labeled antigen, respectively.

For example, when examining occult blood, since the antigen is hemoglobin, the antibody in the labeled antigen or antibody zone is an antihuman hemoglobin antibody, so that the antibody in the confirmation zone is an anti-antihuman hemoglobin antibody which is immunoreactive with the above-mentioned antihuman hemoglobin antibody.

The methods for fixing the antigen or the antibody, the labeled antigen or the labeled antibody, and the antigen or the antibody which is immunoreactive with the labeled antigen or the labeled antibody onto the outer peripheral surface of the rod-shaped core member can be roughly classified into two methods, namely, a method of fixing such antigens or antibodies to the surface of the rod-shaped core member by adsorption (hereinafter referred to as the adsorption method), and a method of bonding such antigens or antibodies to the surface of the rod-shaped core member by chemical bonding (hereinafter referred to as the chemical bonding method).

In the adsorption method, when the rod-shaped core member is made of a plastic material having high adsorption properties, the above-mentioned antigens or antibodies can be directly adsorbed on the outer peripheral surface of the rod-shaped core member, while when the rod-shaped core member is made of a plastic material having low adsorption properties, the outer peripheral surface of the rod-shaped core member is coated with a material which enhances the adsorption properties of the rod-shaped core member, such as nitrocellulose, or tannin, and then the antigen or the antibody is adsorbed on the necessary portions of the adsorption-properties-enhanced peripheral surface of the rod-shaped core member.

The adsorption of the antigen or the antibody by the rod-shaped core member is carried out by the steps of dissolving the antigen or the antibody in a liquid, coating the solution in the shape of a ring band on the necessary portions on the peripheral surface of the rod-shaped core member, and drying the coated solution.

In the chemical bonding method, an active ester applied to the peripheral surface of the rod-shaped core member is allowed to react with an amino group, that is, -NH₂ group, of the antigen or the antibody, and is then reduced, whereby a chemical bond is formed between the antigen or the antibody and the peripheral surface of the rod-shaped core member. Alternatively, a Schiff base is applied to the peripheral surface of the rod-shaped core member, then allowed to react with the amino group of the antigen or the antibody, and then reduced to form a chemical bond between the antigen or the antibody and the peripheral surface of the rod-shaped core member.

By use of any of the above-mentioned methods, the labeled antigen or antibody zone, the detection zone and the confirmation zone are successively formed on the outer peripheral surface of the rod-shaped core member in the direction from the base portion toward the top end portion of the rod-shaped core member.

In order to perform the sucking up of the liquid specimen and to prevent the liquid specimen from penetrating the rod-shaped core member sufficiently, it is preferable that the outer peripheral surface of the rod-shaped core member except the labeled antigen or antibody zone, the detection zone and the confirmation zone be coated with a protein such as albumin, or casein of milk.

In the instrument for simplified immunoassay of the present invention, the cross section of the rod-shaped core member at right angles with respect to the longitudinal direction thereof may be in any shape, such as a circle, an ellipse, and an n-sided polygon (wherein n≥ 3).

For example, when the rod-shaped core member is made so as to have an ellipse-shaped cross section at right angles with respect to the longitudinal direction thereof, and the rod-shaped core member bears thereon two of the labeled antigen or antibody zones, with one of the labeled antigen or antibody zones being provided on a front side of the rod-shaped core member with respect to the major axis of the ellipse, and the other labeled antigen or antibody zone being provided on a back side of the rod-shaped core member with respect to the major axis of the ellipse, and each of the labeled antigen or antibody zones being capable of recognizing a different antigen or a different antibody, with respect to one specimen, two examination items can be assayed or measured simultaneously.

Furthermore, when the rod-shaped core member is made so as to have an n-sided polygon-shaped cross section (wherein n≥ 3) at right angles with respect to the longitudinal direction thereof, and the rod-shaped core member bears on each side thereof one of n labeled antigen or antibody zones, with each of the labeled antigen or antibody zones being capable of recognizing a different antigen or a different antibody, with respect to one specimen, n examination items can be assayed or measured simultaneously.

In the above, it is preferable that the position of each of the labeled antigen or antibody zones be shifted from each other to facilitate the assay.

Furthermore, regardless of the shape of the cross section of the rod-shaped core member, the rod-shaped core member may comprise a plurality of the labeled antigen or antibody zones with an appropriate space therebetween, each of the labeled antigen or antibody zones being capable of recognizing a different antigen or a different antibody, and a corresponding number of the detection zones may be provided so as to correspond to the labeled antigen or antibody zones, whereby a plurality of examination items can be assayed or measured simultaneously.

Furthermore, the labeled antigen or antibody zone may be a single zone which includes a plurality of labeled antigens or labeled antibodies.

Therefore, for instance, when three examination items have to be checked with respect to one specimen, three labeled antigen or antibody zones, each zone comprising a different labeled antigen or a different labeled antibody, are provided, and the corresponding three detection zones, each detection zone comprising an antigen or an antibody which reacts only with the above-mentioned different labeled antigen or labeled antibody, are provided with a predetermined space therebetween.

Furthermore, the instrument for simplified immunoassay of the present invention may further comprise a liquid-suck-up member for sucking up the liquid specimen at the top end portion of the rod-shaped core member.

The instrument for simplified immunoassay of the cover member may further comprise at least one fin member for preventing the rotation of the instrument.

The present invention is also directed to a simplified immunoassay method using any of the above-mentioned instrument for simplified immunoassay.

Other features of this invention will become apparent in the course of the following description of exemplary embodiments, which are given for illustration of the invention and are not intended to be limiting thereof.

### Example 1

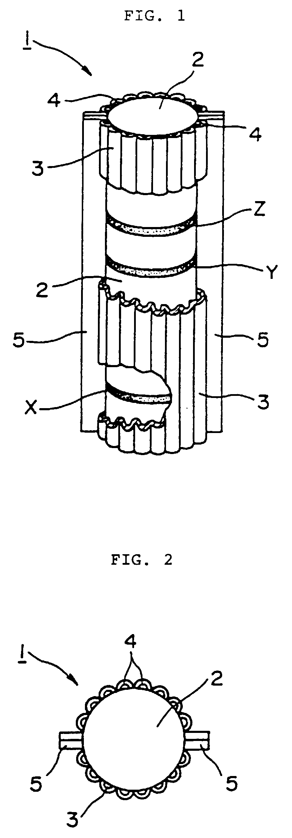

With reference to Fig. 1, a simplified immunoassay instrument 1 of the present invention comprises a core member 2, and a transparent or semitransparent outer cover member 3 which covers the outside of the core member 2.

The core member 2 comprises a rod-shaped member made of polypropylene with a diameter of about 3.0 mm and a length of about 70 mm.

A labeled antigen or labeled antibody zone X (hereinafter simply referred to as "the labeled zone X") in the shape of a ring band is formed so as to encircle the outer peripheral surface of the core member 2 near a base portion thereof.

The labeled zone X is formed by attaching a gold colloid to an antihuman hemoglobin antibody as a labeling material and coating the gold-colloid attached antihuman hemoglobin antibody in the shape of a ring band on the outer peripheral surface of the core member 2 by the above-mentioned adsorption method.

In a portion on the outer peripheral surface of the core member 2, above the labeled zone X, there is formed a detection zone Y in the shape of a ring band encircling the outer peripheral surface of the core member 2.

In the detection zone Y, an anti-hemoglobin antibody which reacts with hemoglobin is coated. Therefore, when hemoglobin is contained in the liquid specimen and the hemoglobin, in a state of being bonded to a labeled antigen or a labeled antibody, reaches the detection zone Y in a large amount, since the labeled antigen or the labeled antibody bears the labeling material thereon which is specifically colored, the presence of the hemoglobin in the specimen can be visually recognized with reference to the color thereof.

In a portion on the outer peripheral surface of the core member 2, above the detection zone Y, there is formed a confirmation zone Z in the shape of a ring band encircling the outer peripheral surface of the core member 2 for confirming the presence of the antigen or the antibody in the detection zone Y.

The confirmation zone Z comprises an anti-antihuman hemoglobin antibody which reacts with a liquid specimen containing the hemoglobin which is bonded to the labeled antigen or the labeled antibody.

With the labeled zone X, the detection zone Y and the confirmation zone Z being formed on the outer peripheral surface of the core member 2 as described above, the remaining uncovered outer peripheral surface portions of the core member 2 are coated with albumin in such a manner that the liquid specimen can be easily and smoothly moved upward along the outer peripheral surface thereof.

The thus prepared core member 2 is then covered with the cover member 3 which is a cylindrical member made of transparent polypropylene.

In the inner surface of the cover member 3, there is formed a plurality of grooves 4 which extends in the direction of the axis of the cover member 3. The non-grooved portions of the inner surface of the cover member 3 are in close or integral contact with the outer peripheral surface of the core member 2 in such a manner that the grooves 4 form capillary channels through which the liquid specimen can move upward by capillary action.

Furthermore, there is provided a pair of fin members 5 on the cover member 3 for preventing the rotation and falling down of the instrument 1. The fin members extend in the longitudinal direction of the cover member 3 as illustrated in Fig. 1.

A method of detecting the presence of hemoglobin in the feces by use of the above-mentioned simplified immunoassay instrument 1 of the present invention will now be explained.

In assaying occult blood in a test sample of the feces, the base portion of the instrument 1, which is a lower portion below the labeled zone X (not specifically shown in Fig. 1), is inserted into the test sample of the feces.

When the test sample of the feces is dry hard, it is dissolved in an eluent comprising physiological saline to prepare a liquid specimen, the base portion of the immunoassay instrument 1 is immersed into the thus prepared liquid specimen. Alternatively, the sample of the feces may be coated on the base portion of the immunoassay instrument 1, and the sample coated base portion may be immersed into the above-mentioned eluent to dissolve the sample therein to prepare a liquid specimen.

By inserting the base portion of the instrument 1 into the specimen, the liquid specimen, that is, an aqueous liquid containing hemoglobin is sucked upward through the grooves 4 between the core member 2 and the cover member 3 by capillary action and then reaches the labeled zone X.

When hemoglobin is contained in the liquid specimen, the hemoglobin is bonded to the antibody labeled with metal colloid particles, moves upward along the core member 2 by capillary action and then reaches the detection zone Y.

When the liquid specimen led together with the antibody labeled with the metal colloid (hereinafter referred to as the metal-colloid-labeled antibody) reaches the detection zone Y, the liquid specimen bonded to the metal-colloid-labeled antibody reacts with the antigen or antibody in the occult blood for examination, and when hemoglobin is contained in the liquid specimen, a large amount of the labeled material gathers. As a result, a marker with a particular color appears in the detection zone Y.

In the absence of hemoglobin in the liquid specimen, no hemoglobin is bonded to the metal-colloid-labeled antibody so that there is no reaction in the detection zone Y. As a result, no marker appears.

When the liquid specimen has reached the detection zone Y, the liquid specimen is further sucked up to a confirmation zone Z.

In the confirmation zone Z, an antigen or an antibody (in this example, an anti-antihuman hemoglobin antibody) which can recognize the labeled antigen or the labeled antibody is attached thereto in the form of a ring band, so that when the liquid specimen comes into contact with the confirmation zone Z, and the labeled antigen or the labeled antibody has activity, a marker appears due to the reaction, whereby it can be confirmed that the liquid specimen has surely passed through the detection zone Y.

Therefore, when the confirmation zone Z is formed and hemoglobin is contained in the liquid specimen, a marker appears in the detection zone Y, and another marker appears in the confirmation zone Z. When no hemoglobin is contained in the liquid specimen, a marker appears only in the confirmation zone Z.

### Example 2

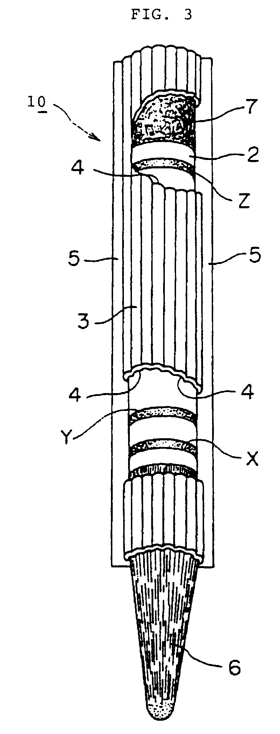

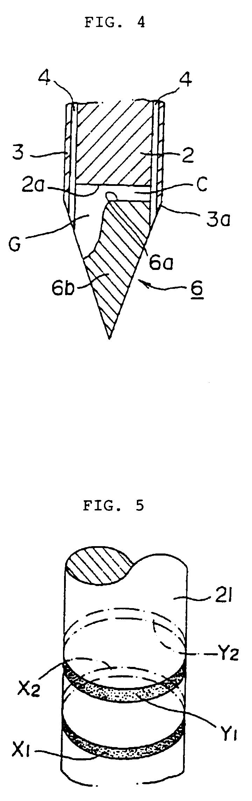

With reference to Figs. 3 and 4, another example of a simplified immunoassay instrument 10 of the present invention will now be explained.

In Figs. 3 and 4, the same references as in Figs. 1 and 2 are used with respect to the identical, substantially identical or corresponding parts in Figs. 1 and 2.

The simplified immunoassay instrument 10 comprises a core member 2, a transparent outer cover member 3 which covers the outside of the core member 2, and a specimen collecting portion 6 which is made of a water-absorbing material.

The core member 2 comprises a circular cylinder made of polypropylene with a diameter of about 3.0 mm and a length of about 60 mm. A labeled antigen or labeled antibody zone X (hereinafter simply referred to as "the labeled zone X") in the shape of a ring band is formed so as to encircle the outer peripheral surface of the circular cylinder near a base portion of the circular cylinder, that is, about 5 mm from a base end 2a thereof which is shown in Fig. 4.

The labeled zone X is formed by attaching a gold colloid as a labeling material to an antihuman hemoglobin antibody in the shape of a ring band on the outer peripheral surface of the core member 2 by the above-mentioned absorption method.

In a portion on the outer peripheral surface of the core member 2, above the labeled zone X, specifically about 10 mm from the base end 2a of the core member 2, there is formed a detection zone Y in the shape of a ring band encircling the outer peripheral surface of the core member 2.

In the detection zone Y, an anti-hemoglobin antibody which reacts with hemoglobin is coated. Therefore, when hemoglobin is contained in the liquid specimen and that hemoglobin, in a state of being bonded to a labeled antigen or a labeled antibody, reaches the detection zone Y in a large amount, since the labeled antigen or antibody bears the labeling thereon which is specifically colored, the presence of the hemoglobin in the specimen can be visually recognized due to the color thereof.

In a portion on the outer peripheral surface of the core member 2, above the detection zone Y, specifically about 50 mm from the base end 2a of the core member 2, there is formed a confirmation zone Z in the shape of a ring band encircling the outer peripheral surface of the core member 2.

The confirmation zone Z comprises an anti-antihuman hemoglobin antibody which reacts with a liquid specimen containing the hemoglobin bonded to the labeled antigen or labeled antibody.

With the labeled zone X, the detection zone Y and the confirmation zone Z being formed on the outer peripheral surface of the core member 2 as described above, the remaining uncovered outer peripheral surface portions of the core member 2 are coated with albumin in such a manner that the liquid specimen can be easily and smoothly moved upward along the outer peripheral surface thereof.

The thus prepared core member 2 is then covered with the cylindrical cover member 3. The cover member 3 is longer than the core member 2 and is made of transparent polypropylene.

In the inner surface of the cylindrical cover member 3, there is formed a plurality of grooves 4 which extend in the direction of the axis of the cylindrical cover member 3 and the undepressed portions other than the grooves 4 at the inner surface of the cylindrical cover member 3 are in close or integral contact with the outer peripheral surface of the core member 2, in such a manner that the grooves 4 form capillary channels through which the liquid specimen can move upward by capillary action.

A tip portion 3a of the cylindrical cover member 3 extends beyond the base end 2a of the core member 2 by a length of about 5 mm.

Furthermore, there is proved a pair of fin members 5 on the cover member 3 for stopping the rotation of the instrument 10 which leads to ready falling down thereof, which extends in the longitudinal direction of the cover member 3 as illustrated in Fig. 3.

At the top end portion of the core member 2, there is formed a water-suck-up member 7 made of a water absorbing material for sucking up the liquid specimen efficiently.

The specimen collecting portion 6 is, for example, a water-absorbing felt member in the shape of a reversed circular cone, having a base end 6a thereof with a diameter of about 3 mm, which is the same as the diameter of the base end 2a of the core member 2, and a height of about 5 mm.

With reference to Fig. 4, the specimen collecting portion 6 is fitted into a space G which is formed by the surface of the base end 2a of the core member 2 and the inner peripheral surface of the tip end portion 3a of the cover member 3 which extends beyond the base end 2a of the core member 2, in such a manner that a gap C is left between the base end 6a of the specimen collecting portion 6 and the base end 2a of the core member 2. Due to the gap C, no capillary portions are formed in the grooves at the inner surface of the cover member 3 in the space G.

A tip portion 6b of the specimen collecting portion 6 is extended from the tip end portion 3a of the cover member 3. However when the tip portion 6b of the specimen collecting portion 6 is depressed in the direction of the space G, the specimen collecting portion 6 is caused to enter the space G and the base end portion 6a of the specimen collection portion 6 comes into contact with the base end 2a of the core member 2, whereby a capillary portion is also formed between the grooves 4 at the inner surface of the cover member 3 and the outer peripheral portion of the specimen collecting portion 6, so that the liquid specimen absorbed by the specimen collecting portion 6 is sucked upward along the peripheral surface of the specimen collecting portion 6 in contact with the grooves 4 at the inner surface of the cover member 3, and then along the outer peripheral surface of the core member 2 which is also in contact with the grooves 4 at the inner surface of the cover member 3.

Even if the gap C is still left between the base end 6a of the specimen collecting portion 6 and the base end 2a of the core member 2, when the gap C is filled with an eluent by immersing the tip portion of the specimen collecting portion 6 into the eluent, the liquid specimen collected by the specimen collecting portion 6 can be sucked upward along the core member 2 by capillary action.

A method of examining the hemoglobin in the feces by use of the above-mentioned simplified immunoassay instrument 10 of the present invention will now be explained.

In assaying occult blood in the feces, when the tip portion 6b of the specimen collecting portion 6 of the immunoassay instrument 1 is inserted into a test sample of the feces, the liquid component in the feces is absorbed by the tip portion 6b. However, due to the presence of the gap C between the base end 2a of the core member 2, with the outer peripheral surface thereof being covered with the cover member 3, and the base end 6a of the specimen collecting portion 6, the grooves 4 at the inner surface of the cover member 3, which form a capillary portion between the inner surface of the cover member 3 and the outer peripheral surface of the core member 2, do not perform capillary action, and the liquid specimen in the faces is merely absorbed by the specimen collecting portion 6. Furthermore, the amount of the liquid specimen absorbed by the specimen collecting portion 6 is a constant amount which is determined by the size of the specimen collecting portion 6. No liquid specimen in an amount which exceeds the constant amount is absorbed by the specimen collecting portion 6.

When a collected sample of the feces is dry hard, it is dissolved in an eluent comprising physiological saline to prepare a liquid specimen, the tip portion 6b of the specimen collecting portion 6 of the immunoassay instrument 10 may be inserted into the thus prepared liquid specimen. Alternatively, the sample of the feces may be coated on the tip portion 6b of the specimen collecting portion 6 of the immunoassay instrument 1, and the sample coated tip portion 6b may be immersed into the above-mentioned eluent to dissolve the sample therein to prepare a liquid specimen.

When a predetermined amount of the liquid specimen is collected by the specimen collecting portion 6, which is an aqueous liquid containing hemoglobin contained in the feces, the specimen collecting portion 6 is immersed into the eluent and at the same time, the tip portion 6b of the specimen collecting portion 6 of the immunoassay instrument 1 is brought into pressure contact with a bottom of a container for the eluent (not shown), whereby the tip portion 6b is caused to enter the inside of the cover member 3, and the specimen collecting portion 6 is connected with the base end 2a of the core member 2. Since the specimen collecting portion 6 is thus connected to the base end 2a of the core member 2, the liquid specimen which is absorbed by the specimen collecting portion 6 is sucked upward through the grooves 4 between the core member 2 and the cover member 3 by capillary action and then reaches the labeled zone X.

At that moment, impurities such as non-specific materials contained in the liquid specimen are absorbed by the specimen collecting portion 6, so that only a liquid specimen free of such impurities reaches the labeled zone X.

When hemoglobin is contained in the liquid specimen, the hemoglobin is bonded to the labeled antibody marked with metal colloid particles, moves upward along the core member 2 by capillary action and then reaches the detection zone Y.

When the liquid specimen led together with the antibody labeled with the metal colloid (hereinafter referred to as the metal-colloid-labeled antibody) reaches the detection zone Y, the liquid specimen bonded to the metal-colloid-labeled antibody reacts with the antigen or antibody in the occult blood for examination, and when hemoglobin is contained in the liquid specimen, a large amount of the labeled material gathers. As a result, a marker with a particular color appears in the detection zone Y.

In the absence of hemoglobin in the liquid specimen, no hemoglobin is bonded to the metal-colloid-labeled antibody so that there is no reaction in the detection zone Y. As a result, no marker appears.

When the liquid specimen has reached the detection zone Y, the liquid specimen is further sucked up by a suck-up member 7 comprising an absorbent pat having stronger capillary action than the capillaries formed between the cover member 3 and the core member 2, and then reaches a confirmation zone Z.

In the confirmation zone Z, an antigen or an antibody (in this example, an anti-antihuman hemoglobin antibody) which can recognize the labeled antigen or the labeled antibody is secured thereto in the form of a ring band, so that when the liquid specimen comes into contact with the confirmation zone Z, and the labeled antigen or the labeled antibody has activity, a marker appears due to the reaction, whereby it can be confirmed that the liquid specimen has surely passed through the detection zone Y.

Therefore, when the confirmation zone Z is formed and hemoglobin is contained in the liquid specimen, a marker appears in the detection zone Y, and another marker appears in the confirmation zone Z. When no hemoglobin is contained in the liquid specimen, a marker appears only in the confirmation zone Z.

When saliva is a specimen and the presence of an HIV antigen in the saliva is detected, as the labeled antibody in the labeled zone X, an anti-HIV antibody is set, and as the antibody in the confirmation zone Z, an anti-anti HIV antibody is set. Thus, when the HIV antigen is present in the saliva, a marker appears in the detection zone Y and another marker in the confirmation zone Z.

In the above-mentioned example, the assay for occult blood and saliva has been explained.

The simplified immunoassay and the instrument therefor of the present invention can also be used for the diagnosis of HIV, hepatitis B, hepatitis C, and pregnancy. In any of the above assays, the result can be obtained within an extremely short time, about less than 5 minutes, after the specimen collecting portion 6 at the base portion of the instrument 1 is inserted into the liquid specimen. Therefore the immunoassay of the present invention is useful as a simplified immunoassay.

In the above-mentioned instrument 1 as shown in Fig. 3, the core member 2 has a round cross section, and there are provided one labeled zone X, one detection zone Y and one confirmation zone Z.

The cross section of the core member 2 shown in Fig. 1 may be relatively flat oval, which is illustrated in Fig. 5 as a core member 21 with a labeled zone X₁ for recognizing an antigen or antibody contained in the specimen and a detection zone Y₁ corresponding to the labeled zone X₁ on a front side, and a labeled zone X₂ for recognizing an antigen or antibody different from the above-mentioned antigen or antibody contained in the specimen and a detection zone Y₂ corresponding to the labeled zone X₂ on a back side thereof, whereby two examination items can be simultaneously assayed.

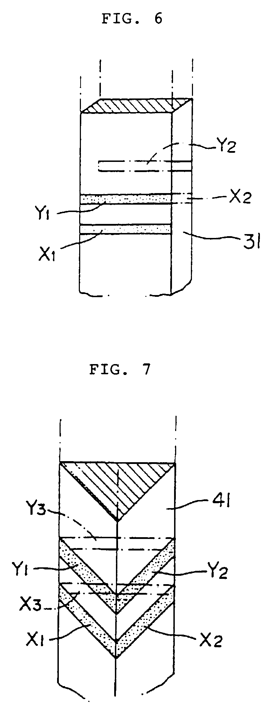

Furthermore, the cross section of the core member 2 as shown in Fig. 3 may be relatively flat rectangular, which is illustrated in Fig. 6 as a core member 31 with a labeled zone X₁ for recognizing an antigen or antibody contained in the specimen and a detection zone Y₁ corresponding to the labeled zone X₁ on a front side, and a labeled zone X₂ for recognizing an antigen or antibody different from the above-mentioned antigen or antibody contained in the specimen and a detection zone Y₂ corresponding to the labeled zone X₂ on a back side thereof, whereby two examination items can be simultaneously assayed.

Alternatively, the cross section of the core member 2 as shown in Fig. 3 may be triangular, which is illustrated in Fig. 7 as a core member 41 with a labeled zone X₁ for recognizing an antigen or antibody contained in the specimen and a detection zone Y₁ corresponding to the labeled zone X₁ on a first side of the core member 41, with a labeled zone X₂ for recognizing an antigen or antibody different from the above-mentioned antigen or antibody contained in the specimen and a detection zone Y₂ corresponding to the labeled zone X₂ on a second side thereof, and with a labeled zone X₃ for recognizing a further antigen or antibody different from the above-mentioned antigen or antibody contained in the specimen and a detection zone Y₃ corresponding to the labeled zone X₃ on a third side thereof, whereby three examination items can be simultaneously assayed.

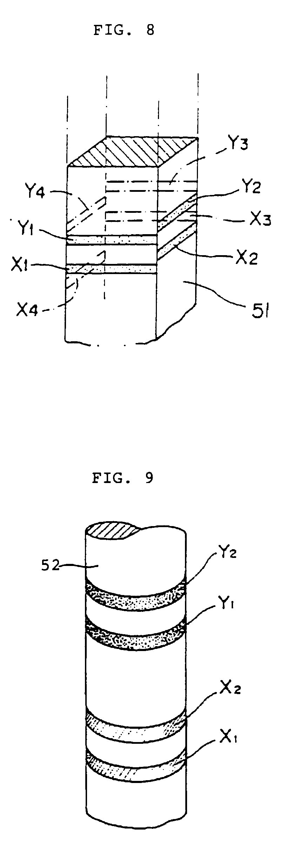

Furthermore, the cross section of the core member 2 as shown in Fig. 3 may be square, which is illustrated in Fig. 8 as a core member 51 with a labeled zone X₁ for recognizing an antigen or antibody contained in the specimen and a detection zone Y₁ corresponding to the labeled zone X₁ on a first side of the core member 51, with a labeled zone X₂ for recognizing an antigen or antibody different from the above-mentioned antigen or antibody contained in the specimen and a detection zone Y₂ corresponding to the labeled zone X₂ on a second side thereof, with a labeled zone X₃ for recognizing a further antigen or antibody different from the above-mentioned antigen or antibody contained in the specimen and a detection zone Y₃ corresponding to the labeled zone X₃ on a third side thereof, and with a labeled zone X₄ for recognizing still another antigen or antibody different from the above-mentioned antigen or antibody contained in the specimen and a detection zone Y₄ corresponding to the labeled zone X₄ on a fourth side thereof, whereby four examination items can be simultaneously assayed.

Furthermore, the cross section of the core member 2 as shown in Fig. 3 may be in any shape.

Specifically as illustrated in Fig. 9, regardless of the shape of the cross section of a core member 52, for example, there can be formed a labeled zone X₁ for recognizing an antigen or antibody contained in the specimen, a labeled zone X₂ for recognizing an antigen or antibody different from the above-mentioned antigen or antibody contained in the specimen, a detection zone Y₁ corresponding to the labeled zone X₁ and a detection zone Y₂ corresponding to the labeled zone X₂, whereby a plurality of examination items can be simultaneously assayed.

In the conventional paper chromatography, the sensitivity thereof increases in accordance with the evaporation of the liquid specimen, so that it has a problem that it is extremely difficult to set a cut-off value for the assay. However, according to the present invention, the liquid specimen is sucked up through the capillary portion and also covered with the cover member, so that it is unnecessary to take care of the prevention of the evaporation of the liquid specimen during the examination.

Furthermore, according to the present invention, the changes in the sensitivity over the examination time and day are extremely small, and therefore the presence or absence of the antigen or the antibody can be most accurately detected and confirmed.

Furthermore, according to the present invention, specimens which are difficult to examine by the conventional methods due to the presence of a large amount of solid components and non-specific components, such as saliva and expectoration, can be easily examined, so that the present invention will find many applications.

## Claims

1. An instrument for simplified immunoassay (1) for detecting the presence of at least one of an antigen or an antibody in a liquid specimen, comprising:
a rod-shaped core member (2) with a base portion and a top end portion, there being successively formed on the outer peripheral surface thereof, in the direction from said base portion toward said top end portion, (a) at least one labeled antigen or antibody zone (X) comprising a labeled antigen or a labeled antibody, said labeled antigen being capable of reacting with said antibody in said liquid specimen, and said labeled antibody being capable of reacting with said antigen in said liquid specimen, each forming a labeled antigen-antibody complex, and (b) a detection zone (Y) which is disposed above said labeled antigen or antibody zone (X) on the outer peripheral surface of said rod-shaped core member (2), said detection zone (Y) comprising an antigen or an antibody capable of reacting with said labeled antigen-antibody complex, and
a cover member (3) which covers the outer peripheral surface of said rod-shaped core member (2), through which said labeled antigen or antibody zone (X) and said detection zone (Y) formed on the outer peripheral surface of said rod-shaped core member (2) are visible, with a capillary portion being provided between said rod-shaped core member (2) and said cover member (3), through which capillary portion said liquid specimen is carried upward in the direction from said base portion to said top end portion so as to have said liquid specimen reach said labeled antigen or antibody zone (X) and then said detection zone (Y).

2. The instrument for simplified immunoassay (10) as claimed in Claim 1, further comprising a liquid specimen collection portion (6) for collecting said liquid specimen so as to carry said liquid specimen upward through said capillary portion from said base portion of said rod-shaped core member (2) along its peripheral surface.

3. The instrument for simplified immunoassay as claimed in Claim 1 or 2, wherein said capillary portion comprises a plurality of grooves (4) formed along the inner surface of said cover member (3), which extends in the direction from said base portion to said top end portion of said rod-shaped core member (2).

4. The instrument for simplified immunoassay as claimed in any one of Claims 1 to 3, further comprising a liquid-suck-up member (7) for sucking up said liquid specimen, which is disposed in said top end portion of said rod-shaped core member (2).

5. The instrument for simplified immunoassay as claimed in any one of Claims 1 to 4, wherein the outer peripheral surface of said rod-shaped core member (2) except said labeled antigen or antibody zone (X) and said detection zone (Y) is coated with a protein.

6. The instrument for simplified immunoassay as claimed in any one of Claims 1 to 5, further comprising a confirmation zone (Z) above said detection zone (Y) on the outer peripheral surface of said rod-shaped core member (2), said confirmation zone (Z) comprising an antigen or an antibody capable of recognizing said labeled antigen or said labeled antibody and being visible through said cover member (3).

7. The instrument for simplified immunoassay as claimed in any one of Claims 2 to 6, wherein said cover member (3) extends downward beyond said base portion of said rod-shaped core member (2) to form a space (G) under said base portion of said rod-shaped core member (2), surrounded by said cover member (3), and said liquid specimen collecting portion (6) comprises a water-absorbing material, which is movably fitted into said space (G) with a predetermined gap (C) between said base portion of said rod-shaped core member (2) and a base end portion (2a) of said liquid specimen collecting portion (6), and can be connected with the base portion of said rod-shaped core member (2) when depressed so as to be fitted into said space (G), said liquid specimen being thereby carried from said liquid specimen collection portion (6) upward through said capillary portion along said peripheral surface of said rod-shaped core member (2).

8. The instrument for simplified immunoassay as claimed in any one of Claims 1 to 7, wherein the cross section of said rod-shaped core member (2) at right angles with respect to the longitudinal direction thereof is in the shape of a circle.

9. The instrument for simplified immunoassay as claimed in any one of Claims 1 to 7, wherein the cross section of said rod-shaped core member (2) at right angles with respect to the longitudinal direction thereof is in the shape of an ellipse, and said rod-shaped core member (2) bears thereon two labeled antigen or antibody zones (X₁, X₂), and one of said labeled antigen or antibody zones (X₁, X₂) is provided on a front side of said rod-shaped core member (2) with respect to the major axis of the ellipse, and the other labeled antigen or antibody zone (X₁, X₂) is provided on a back side of said rod-shaped core member (2) with respect to the major axis of the ellipse, each of said labeled antigen or antibody zones (X₁, X₂) being capable of recognizing a different antigen or a different antibody.

10. The instrument for simplified immunoassay as claimed in any one of Claims 1 to 7, wherein the cross section of said rod-shaped core member (2) at right angles with respect to the longitudinal direction thereof is in the shape of an n-sided polygon (wherein n ≥ 3), and said rod-shaped core member (2) bears on each side thereof one of n of said labeled antigen or antibody zones (X), each of said labeled antigen or antibody zones (X) being capable of recognizing a different antigen or a different antibody.

11. The instrument for simplified immunoassay as claimed in any one of Claims 1 to 7, wherein said rod-shaped core member (2) comprises a plurality of said labeled antigen or antibody zones (X), each of said labeled antigen or antibody zones (X) being capable of recognizing a different antigen or a different antibody, and a plurality of said detection zones (Y) is provided so as to correspond to said labeled antigen or antibody zones (X).

12. The instrument for simplified immunoassay as claimed in any one of Claims 1 to 11, wherein said rod-shaped core member (2) is made of a synthetic resin.

13. The instrument for simplified immunoassay as claimed in any one of Claims 1 to 12, wherein said cover member (3) is transparent or semitransparent.

14. The instrument for simplified immunoassay as claimed in any one of claims 1 to 13, wherein said cover member further comprises at least one fin member (5) for preventing the rotation of said instrument.

15. The instrument for simplified immunoassay as claimed in any one of Claims 7 to 14, wherein said water-absorbing material for said specimen collecting portion (6) is selected from the group consisting of an open-cell sponge material and a felt.

16. A simplified immunoassay method using an instrument for simplified immunoassay for detecting the presence of at least one of an antigen or an antibody in a liquid specimen, wherein said instrument is as claimed in any one of claims 1 to 15.

17. The simplified immunoassay method as claimed in Claim 16, wherein said labeled antigen and said labeled antibody are respectively labeled with a labeling material selected from the group consisting of a metal colloid, a color latex and an organic coloring material.
